# EUROPEAN PATENT APPLICATION

(11) **EP 4 620 421 A1**
(43) Date of publication of application: **24.09.2025**
(21) Application number: 25164314.4
(22) Date of filing: 18.03.2025
(51) Int. Cl.: A61B 34/20, A61B 34/10, A61B 90/00

(54) **AUTOMATIC RECENTERING OF SURGICAL NAVIGATION VIEW ON INSTRUMENT TIP**

(30) Priority: 19.03.2024 US 202463567284 P
(71) Applicant: Stryker European Operations Limited, Carrigtwohill, Co. Cork T45 HX08 (IE)
(72) Inventor: MARTINS, Maria Manuel Carvalho de Freitas, 68420 Eguisheim (FR); KUMAR, Pankaj, 110005 New Delhi (IN)
(74) Representative: Röthinger, Rainer

(57) **Abstract**

A surgical navigation system including an instrument, a localizer, a memory device, a display, and a controller is provided. The memory device contains at least one slice image and a volumetric image defined relative to a known coordinate system. The controller is configured to show a view of the at least one slice image which corresponds to a first sub-volume of the volumetric image on the display, determine a position of the instrument relative to the known coordinate system, determine a movement parameter of the instrument, determine that the view of the first sub-volume should no longer be shown on the display, determine a second sub-volume of the volumetric image based on the position of the instrument relative to the known coordinate system, and show a view of the at least one slice image which corresponds to the second sub-volume of the volumetric image on the display.

## Description

### CROSS-REFERENCE TO RELATED APPLICATIONS

The present application claims priority to and all the benefits of U.S. Provisional Patent Application No. 63/567,284, filed on March 19, 2024, the entire contents of which are expressly incorporated herein by reference.

### BACKGROUND

Surgical navigation systems are often used to track and display the position of surgical instruments relative to a patient during surgical procedures. Many of these systems include at least one display upon which images of, or representing, anatomy of the patient are shown. A user, such as a surgeon, may refer to the display in order to determine where the surgical instrument is relative to the patient anatomy. This can be especially useful during operations on sensitive elements of the anatomy of the patient, such as the spine of the patient, and/or during operations that involve inserting surgical instruments inside of the patient such that the position of the instrument cannot be seen from outside of the patient.

During surgical operations, the user may move the instrument to a new position relative to the patient anatomy, and the surgical navigation system may update the display to show the instrument in the new position. However, there are scenarios where the user wants the display to show the patient anatomy in a fixed manner, which may cause the instrument to be shown near the edge of the display if the instrument is positioned near anatomy which is being shown near the edge of the display. This may even cause the instrument not to be shown anymore if the instrument is positioned near anatomy which is outside of the displayed portion of the patient anatomy. As a result, it can be advantageous to provide a surgical navigation system which can recenter the display on the patient anatomy which is near the new position of the instrument.

### SUMMARY

According to a first aspect, a surgical navigation system is provided. The surgical navigation system includes an instrument, a localizer configured to track the position of the instrument, a memory device, a display, and a controller in communication with the localizer, the display, and the memory device. The memory device contains a volumetric image defined relative to a known coordinate system and including a width along an x-axis of the known coordinate system, a depth along a y-axis of the known coordinate system, and a height along a z-axis of the known coordinate system. The controller is configured to show a view of the at least one slice image which corresponds to a first sub-volume of the volumetric image on the display, determine a position of the instrument relative to the known coordinate system, determine a movement parameter of the instrument, determine that the view of the at least one slice image which corresponds to the first sub-volume should no longer be shown on the display based on the movement parameter of the instrument and the position of the instrument relative to the known coordinate system, determine a second sub-volume of the volumetric image based on the position of the instrument relative to the known coordinate system, and show a view of the at least one slice image which corresponds to the second sub-volume of the volumetric image on the display.

According to a second aspect, a computer-implemented method of displaying an instrument relative to a volumetric image of a patient anatomy on a display is provided. The computer-implemented method includes accessing the volumetric image from a memory device, the volumetric image being defined relative to a known coordinate system, and accessing at least one slice image from the memory device. Subsequently, the method includes controlling the display to show a view of the at least one slice image which corresponds to a first sub-volume of the volumetric image on the display, determining a position of the instrument relative to the known coordinate system, determining a movement parameter of the instrument, determine that the view of the at least one slice image which corresponds to the first sub-volume should no longer be shown on the display based on the movement parameter of the instrument and the position of the instrument relative to the known coordinate system, determining a second sub-volume of the volumetric image based on the position of the instrument relative to the known coordinate system, and controlling the display to show a view of the at least one slice image which corresponds to the second sub-volume of the volumetric image on the display.

According to a third aspect, a non-transitory computer-readable medium (or computer program product) is provided comprising instructions, which when executed by one or more processors, are configured to: access a volumetric image from a memory device, the volumetric image being defined relative to a known coordinate system; access at least one slice image from the memory device, the at least one slice image being derived from the volumetric image; control a display to show a view of the at least one slice image which corresponds to a first sub-volume of the volumetric image on the display; determine a position of an instrument relative to the known coordinate system; determine a movement parameter of the instrument; determine that the view of the at least one slice image which corresponds to the first sub-volume should no longer be shown on the display based on the movement parameter of the instrument and the position of the instrument relative to the known coordinate system; determine a second sub-volume of the volumetric image based on the position of the instrument relative to the known coordinate system; and control the display to show a view of the at least one slice image which corresponds to the second sub-volume of the volumetric image on the display.

According to a fourth aspect, a surgical navigation system is provided. The surgical navigation system includes an instrument, a localizer configured to track the position of the instrument, a display, and a controller. The surgical system optionally includes a memory device containing at least one slice image, and a volumetric image defined relative to a known coordinate system and including a width along an x-axis of the known coordinate system, a depth along a y-axis of the known coordinate system, and a height along a z-axis of the known coordinate system. The controller may be in communication with at least one of the localizer, the display, and the memory device. Further, the controller is configured to show a view of a slice image which corresponds to a first sub-volume of a volumetric image on the display, determine a position of the instrument relative to the known coordinate system, and determine a movement parameter of the instrument. Based on the movement parameter of the instrument and the position of the instrument relative to the known coordinate system, the controller is also configured to determine whether the view of the first sub-volume should be shown on the display. If the view should not be shown, the controller may determine a second sub-volume of the volumetric image based on the position of the instrument relative to the known coordinate system and show a view of the at least one slice image which corresponds to the second sub-volume of the volumetric image on the display.

According to a fifth aspect, a surgical navigation system is provided. The surgical navigation system includes a localizer configured to track the position of an instrument, a memory device, a display, and a controller in communication with the localizer, the display, and the memory device. The memory device contains at least one slice image, and a volumetric image defined relative to a known coordinate system and including a width along an x-axis of the known coordinate system, a depth along a y-axis of the known coordinate system, and a height along a z-axis of the known coordinate system. The controller is configured to show a view of the at least one slice image which corresponds to a first sub-volume of the volumetric image on the display, determine a position of the instrument relative to the known coordinate system, determine a movement parameter of the instrument, determine that the view of the at least one slice image which corresponds to the first sub-volume should no longer be shown on the display based on the movement parameter of the instrument and the position of the instrument relative to the known coordinate system, determine a second sub-volume of the volumetric image based on the position of the instrument relative to the known coordinate system, and show a view of the at least one slice image which corresponds to the second sub-volume of the volumetric image on the display.

According to a sixth aspect, a surgical navigation system is provided comprising: a localizer configured to track a position of an instrument relative to an anatomy; a memory comprising a computer-generated 3D model of the anatomy; a display; and a controller in communication with the localizer, the memory, and the display and the controller configured to: present, on the display, a graphical representation of the instrument based on the tracked position of the instrument; present, on the display, a first 2D view derived from the computer-generated 3D model and the graphical representation of the instrument relative to the first 2D view; establish a virtual buffer zone relative to a periphery of the first 2D view; detect that the graphical representation of the instrument has moved into the virtual buffer zone; and in response to detection of the instrument in the virtual buffer zone, present, on the display, a second 2D view derived from the computer-generated 3D model. Also provided are a non-transitory computer readable medium (or computer program product) for any of the above aspects.

Any of the above aspects can be combined in part or in whole with any other aspect. Any of the above aspects, whether combined in part or in whole, can be further combined with any of the following implementations, in full or in part.

In some implementations, the first sub-volume is defined between a first set of coordinate bounds. The first set of coordinate bounds may include a minimum x-value coordinate, a minimum y-value coordinate, a minimum z-value coordinate, a maximum x-value coordinate, a maximum y-value coordinate, and a maximum z-value coordinate. Since the first sub-volume is a part of the volumetric image, the distance between the minimum x-value and the maximum x-value may be less than the width of the volumetric image, the distance between the minimum y-value and the maximum y-value may be less than the depth of the volumetric image, and the distance between the minimum z-value and the maximum z-value may be less than the height of the volumetric image.

In some implementations, the controller is further configured to show the view of the at least one slice image which corresponds to the second sub-volume of the volumetric image based on the movement parameter of the instrument and coordinate bounds of the first sub-volume. The controller may be configured to determine a distance between the position of the instrument and at least one coordinate of the set of coordinate bounds. Further, the controller may be further configured to show the view of the at least one slice image which corresponds to the second sub-volume of the volumetric image based on the movement parameter of the instrument and the distance between the position of the instrument and at least one coordinate of the set of coordinate bounds. Even further, the controller may be configured to compare the distance between the position of the instrument and at least one coordinate of the set of coordinate bounds against a threshold distance, and show the view of the at least one slice image which corresponds to the second sub-volume of the volumetric image based on the movement parameter of the instrument and the comparison of the distance between the position of the instrument and at least one coordinate of the set of coordinate bounds and the threshold distance. Even further, the controller may be configured to determine that the position of the instrument is outside of the first sub-volume, compare the distance between the position of the instrument and at least one coordinate of the set of coordinate bounds against an exterior threshold distance, and show the view of the at least one slice image which corresponds to the second sub-volume of the volumetric image based on the movement parameter of the instrument and the distance between the position of the instrument and at least one coordinate of the set of coordinate bounds being below the exterior threshold distance. Even further, the controller may be configured to determine that the position of the instrument is inside of the first sub-volume, compare the distance between the position of the instrument and at least one coordinate of the set of coordinate bounds against an interior threshold distance, and show the view of the at least one slice image which corresponds to the second sub-volume of the volumetric image based on the movement parameter of the instrument and the distance between the position of the instrument and at least one coordinate of the set of coordinate bounds being below the interior threshold distance.

In some implementations, the second sub-volume includes determining a second set of coordinate bounds. The second set of coordinate bounds may include a minimum x-value coordinate, a minimum y-value coordinate, a minimum z-value coordinate, a maximum x-value coordinate, a maximum y-value coordinate, and a maximum z-value coordinate. The second set of coordinate bounds may be determined as coordinates offset from a centroid of the second sub-volume by a predetermined distance.

In some implementations, the movement parameter includes at least one of a displacement of the instrument, a speed of the instrument, an acceleration of the instrument, and a velocity of the instrument. The movement may include a displacement of the instrument during a first time period. In such an implementation, the controller may be configured to compare the displacement of the instrument during the first time period to a threshold displacement. Further, the controller may be configured to show the view of the at least one slice image which corresponds to the second sub-volume of the volumetric image on the display based on the position of the instrument relative to the first sub-volume of the volumetric image and the comparison of the displacement of the instrument during the first time period and the threshold displacement.

In some implementations, showing the view of the at least one slice image which corresponds to the second sub-volume of the volumetric image on the display includes determining a perspective reference point in the known coordinate system. Such implementations may include determining a view axis defined as a line between the perspective reference point and a centroid of the second sub-volume, determining which slice image of the at least one slice image corresponds to the view axis, determining which portion of the determined slice image corresponds to the second sub-volume, and showing the determined portion of the determined slice image. Alternatively, in other implementations, showing the view of the at least one slice image which corresponds to the second sub-volume of the volumetric image on the display includes determining a perspective vector in the known coordinate system. Such implementations may further include determining a view axis defined as a line extending from a centroid of the second sub-volume and in the direction of the perspective vector, determining which slice image of the at least one slice image corresponds to the view axis, determining which portion of the determined slice image corresponds to the second sub-volume, and showing the determined portion of the determined slice image.

In some implementations, determining the second sub-volume is based on determining a centroid for the second sub-volume based on the position of the instrument. In such implementations, the controller may be configured to determine the centroid of the second sub-volume as the position of the instrument relative to the known coordinate system as determined by the controller. Alternatively, the controller may be configured to determine the centroid of the second sub-volume as a coordinate point offset from the position of the instrument by a predefined distance. In some implementations, the controller is configured to stop showing the first sub-volume of the volumetric image when the second sub-volume is shown on the display. In some implementations, the at least one slice image includes at least one CT image slice and/or the volumetric image is based on the at least one CT image slice. In some implementations, the position of the instrument is a position of an end effector of the instrument.

The first 2D view can remain static such that it does not change view based on changes in the tracked position of the instrument. The second 2D view is displayed such that the centroid of the second 2D view corresponds to the tracked position of the instrument. The virtual buffer zone can be established relative to the periphery of the first 2D view (window frame) such that an inner buffer zone is defined inside the periphery and an outer buffer zone is defined outside of the periphery. The inner buffer zone can have a thickness of 3 cm and the outer buffer zone can have a thickness of 5 cm, thereby defining a total thickness of 8cm for the virtual buffer zone. The controller can detect that the graphical representation of the instrument has moved into the virtual buffer zone for a threshold amount of time, such as greater than 1, 2 or 3 seconds. In response to detection of the instrument in the virtual buffer zone and for the threshold amount of time, the controller can present, on the display, the second 2D view derived from the computer-generated 3D model. The graphical representation can include a 2D or 3D representation of the instrument, or a portion thereof, such as just the tip of the instrument.

### BRIEF DESCRIPTION OF THE DRAWINGS

Figure 1 is a perspective view of an exemplary layout of an operating room including at least one surgical instrument assembly and a surgical navigation system for performing a medical procedure on a patient, according to the teachings of the present disclosure.
Figure 2 is a schematic of an exemplary graphical user interface of a navigation system, the graphical user interface including various windows.
Figure 3, a flow diagram describing a method of controlling at least one of the windows of the graphical user interface of Figure 2 is shown.
Figure 4 shows an exemplary image of a patient anatomy along with a window frame and buffers corresponding to one of the windows of the graphical user interface of Figure 2.
Figures 5A-5C show the exemplary image of the patient anatomy of Figure 4 along with the window frame and buffers of the graphical user interface of Figure 2 centered on various positions of a surgical instrument relative to the exemplary image.
Figure 6 is a schematic of the exemplary graphical user interface of Figure 2 in which views of the patient anatomy have been updated to align with the window frame of Figure 5B.
Figure 7 is a schematic of the exemplary graphical user interface of Figure 2 in which views of the patient anatomy have been updated to align with the window frame of Figure 5C.

### DETAILED DESCRIPTION

### I. Example System Overview

Referring to the Figures, wherein like numerals indicate like or corresponding parts throughout the several views, a surgical system 100 including a surgical navigation system 110 and methods for using the same are shown throughout.

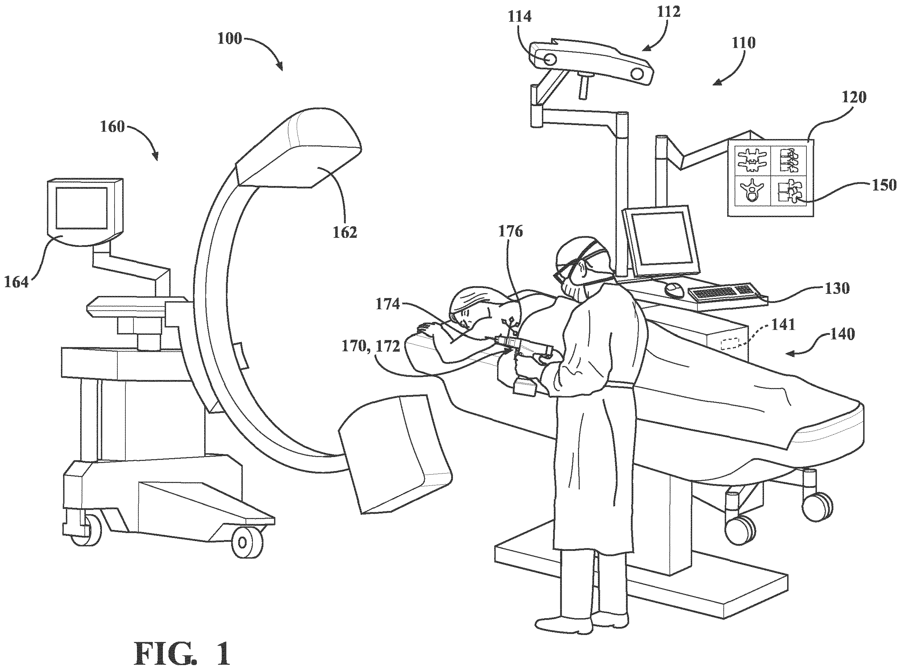

Referring to Figure 1, an exemplary configuration of an operating room or surgical suite for performing a medical procedure on a patient using the surgical system 100 is shown. The surgical navigation system 110 may include a navigation computer 140, a controller 141, user input devices 130, a display 120, and a localizer 112. The navigation computer 140 may include a processor (not shown), memory device (not shown), and storage (not shown). The navigation computer 140 may be a personal computer, laptop computer, tablet computer or any other suitable computing device. The navigation computer 140 may include surgical navigation software including one or more modules and/or operating instructions related to the operation of the surgical navigation system 110 and to implement the various routines, functions, or methods disclosed herein. Further, the controller 141 may be part of the navigation computer 140, or the controller 141 may be implemented by multiple computing devices, such as the navigation computer 140 and a cloud computing device. In some implementations, the controller 141 includes a cloud computing device which communicates with the navigation computer 140 (e.g. the processor of the navigation computer 140).

The display 120 is configured to display various graphical user interfaces (GUI) 150 and patient images (e.g., pre-operative patient images or intraoperative patient images). The pre-operative images may be uploaded to the surgical navigation system 110 prior to the surgical procedure. A user such as a medical professional may interact with the various GUIs 150 via user input devices 130, via touch input, or via other suitable means. The display 120 of the surgical navigation system 110 may be configured to display various prompts or data entry boxes. For example, the display 120 may be configured to display a text box or prompt that allows the user to manually enter or select the type of surgical procedure to be performed.

The display 120 may be further configured to display a surgical plan for a medical procedure overlaid on the patient images. The surgical plan may include the surgical pathway for executing the medical procedure, planned trajectory, orientation, and/or position for the medical instrument and/or implant during the medical procedure. The surgical plan may also include a pose of an implant or medical device to be inserted during the medical procedure overlaid onto the patient data or image. It is contemplated that the surgical navigation system 110 may be configured to display and/or project a holographic image of surgical pathway for executing the medical procedure or planned trajectory or orientation for the medical instrument during the medical procedure. This may include projecting the surgical pathway onto the patient or other surface in the operating room. It may also include a projection of the surgical pathway onto the head unit worn by the user, such as a lens, shield, or glasses of the head unit. An exemplary configuration of the surgical navigation system 110 including a display worn by the user to display the target trajectory and/or target location is disclosed in International Publication No. WO/2018/203304 A1, the entirety of which is hereby incorporated by reference.

In some implementations, the display 120 may be configured as an extended reality device configured to execute any of the graphical functions described herein. The extended reality device can be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user. The real-world views may be views may be those acquired directly by the eyes of the user or may be a real-world video stream captured by one or more cameras of the extended reality device. When a head-mounted device is utilized, the head-mounted device may comprise a transparent lens or one or more display screens positioned directly in front of the eyes of the user to display the computer-generated graphics relative to the real-world views.

The GUI 150 may be configured to allow the user to input or enter patient data or modify the surgical plan. The patient data, in addition to the patient images, may include additional information related to the type of medical procedure being performed, the patient's anatomical features, the patient's specific medical condition, and/or operating settings for the surgical navigation settings. For example, in performing a spinal fusion procedure, the user may enter information via the user input devices 130 and/or the GUI 150 related to the specific vertebra or vertebra on which the medical procedure is being performed. The user may also input various anatomical dimensions related to the vertebrae and/or the size and shape of a medical device or implant to be inserted during the medical procedure. The user input devices 130 and/or the GUI 150 may also be configured to allow the user to select, edit or manipulate the patient data. For example, the user may identify and/or select anatomical features from the patient data. This may include selecting the surgical site, such as selecting the vertebra and/or specific area on the vertebra where the medical procedure is to be performed.

The surgical navigation system 110 may be configured to utilize segmentation to facilitate various features of surgical navigation, such as tool guidance and the generation of alert zones of interests around critical anatomical features. These critical anatomical features may include, cortical walls, nerves, blood vessels or similar critical anatomical structures. The alert zones may be defined by one or more virtual boundaries. The user may also provide input to the user input devices 130 or to the GUI 150 to identify additional critical anatomical features and/or alert zones in addition to what was suggested by the controller 141 or wish to edit alert zones and/or virtual boundaries generated by the controller 141. The user may also provide input to the user input devices 130 or to the GUI 150 to select and/or input a target location, target trajectory, target depth or similar feature of the surgical pathway to help guide the user in performing the medical procedure. The input to the user input devices 130 or to the GUI 150 may be provided to select the surgical instrument to be used, to select the device and/or implant to be inserted, to select a planned pose where the device or implant is to be placed within the patient, and to allow the user to select the parameters of the implant to be inserted, such as the length and/or diameter of the screw to be inserted.

The surgical system 100 may also include an imaging system 160 in communication with the surgical navigation system 110. The imaging system 160, such as CT or MRI imaging device, may perform intraoperative imaging. If the imaging system 160 is a CT imaging device, the imaging system 160 may generate CT image data. The imaging system 160 may include a scanner 162 and an imager display 164. The scanner 162 may be utilized to take an image of the patient and display it on the imager display 164. For example, the scanner 162 may include a C-arm configured to be rotated about the patient to produce a plurality of images of the patient. The imaging system 160 may also include a processor (not shown) including software, as is known by those skilled in the art, which is capable of taking the plurality of images captured by the scanner 162 and producing a two-dimensional image and/or a three-dimensional model of at least a portion of the patient. The imager display 164 may be configured to display the resulting two-dimensional image and/or three-dimensional model.

The imaging system 160 may also be in communication with the controller 141 of the surgical navigation system 110. The imaging system 160 may be configured to communicate via a wired and/or a wireless connection with the controller 141. For example, the imaging system 160 may be configured to provide pre-operative and/or intra-operative image data, such as the resulting 2D image and/or 3D model of the patient, to the controller 141 to provide the resulting 2D image and/or 3D model to the display 120. If the imaging system 160 is a CT imaging device, the imaging system 160 may provide the controller 141 with CT image data.

The surgical system 100 also includes a surgical instrument assembly 170 in wired or wireless communication with the controller 141 directly, or indirectly. While only one surgical instrument assembly 170 is illustrated in Figure 1, the illustrated implementation is only an exemplary configuration of the surgical system 100, and it is contemplated that any number of surgical instrument assemblies may be positioned within the operating room. The surgical instrument assembly 170 includes a surgical instrument 172 including an end-effector 174 and a tracking device 176. Any reference to the surgical instrument 172 should be understood to include any or all of the elements of the surgical instrument assembly 170. The tracking device 176 includes a plurality of markers that are capable of being identified and/or tracked by the surgical navigation system 110. Reliable tracking of surgical instruments during the execution of surgical procedures to follow the planned surgical pathway and/or to avoid critical anatomical structures is of the utmost importance. Furthermore, providing feedback and/or notifying the user executing the procedure when the surgical instrument becomes misaligned with the surgical pathway and/or is at risk of impinging on a critical anatomical structure is of similar importance. The surgical instrument 172 may be coupled to a drill chuck, a tap for creating threads on the interior surface of a hole or aperture, a driver for driving or inserting a screw within the borehole or aperture of the bone, or another end effector. The surgical instrument assembly 170 may each be like any of those described in Intl. Patent Publication No. 2021/062373, which is hereby incorporated by reference in its entirety. The surgical system may, in addition or as an alternative to the surgical instrument assembly 170, include a surgical robot, such as the robotic manipulator described in U.S. Patent No. 11,033,341, which is hereby incorporated by reference.

Further, the navigation system 110 may utilize the localizer 112 to track the instrument assembly 170, the surgical robot, and/or other elements of the surgical system 100. The localizer 112 may include one or more sensors 114 for tracking the tracking device 176 of the surgical instrument assembly 170. The sensors may include cameras, such as CCD cameras, CMOS cameras, and/or optical image cameras, magnetic sensors, radio frequency sensors, or any other sensor adapted to detect and/or sense the position of a tracking device 176 of the surgical instrument assemblies 170. Description of a suitable localizer, and the various localizers that it can utilize may be found in U.S. Patent Publication No. 2017/0333137, which is hereby incorporated by reference in its entirety.

### II. Patient Display Methods

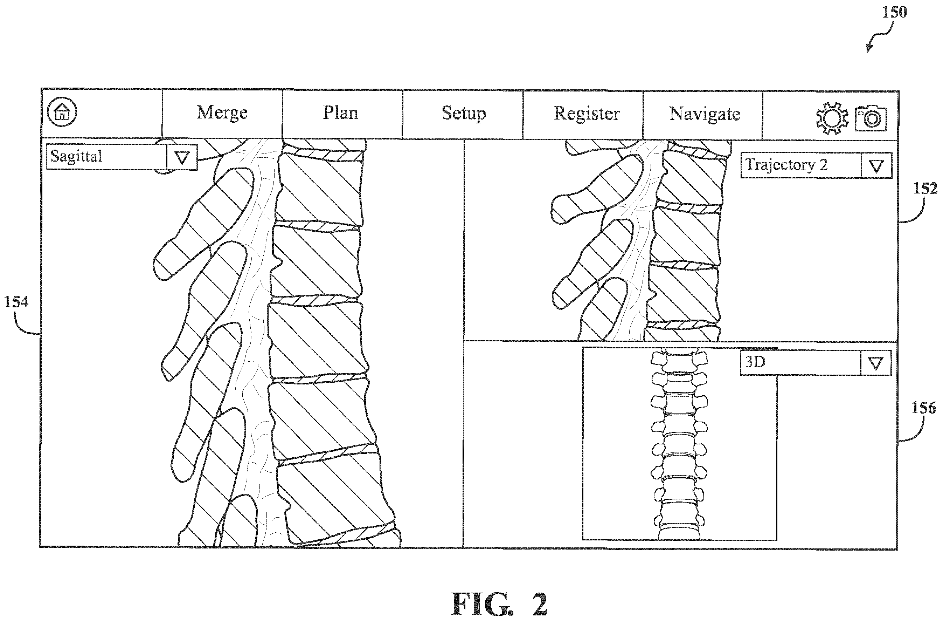

Referring to Figure 2 an exemplary configuration of the graphic user interface (GUI) 150 of the surgical navigation system 110 is illustrated. The GUI 150 may be controlled by the controller 141 and configured as a touch screen on the display 120 of the surgical navigation system 110, an interactable object in a virtual/augmented reality space, or any suitable alternative.

In the illustrated implementation, the GUI 150 includes a first window 152, a second window 154, and a third window 156, each of which contain a view of a patient image. In this implementation, the first window 152 contains a trajectory view of the patient image, the second window 154 contains a sagittal view of the patient image, and the third window 156 contains a perspective view of a 3D model of the patient. The GUI 150 is shown operating in a navigation mode, but the GUI may also operate in other modes, such as a planning mode and a registration mode. The methods and functions of the system 100 are described with reference to the illustrated implementation in which the GUI 150 is operating in the navigation mode, but may also be carried out while the GUI 150 is in the planning mode, the registration mode, or any other mode.

As described above, the display 120 is configured to display patient images (e.g., pre-operative patient images or intraoperative patient images) via the GUI 150. An entirety of the patient image may be shown in the windows 152, 154, 156, but a user may prefer only a portion of the patient image be shown in at least one of the windows 152, 154, 156, This way, the GUI 150 can present a closer look at the patient anatomy. Further, at least part of the surgical instrument 172 may be overlaid onto the patient image in at least one of the windows 152, 154, 156 based on the position of the surgical instrument 172 relative to the patient anatomy. In the illustrated implementation, the end effector 174 of the surgical instrument 172 is overlaid onto the patient image shown in the first window 152. Showing only a portion of the patient image in the first window 152 also provides a closer look at a position of the end effector 174 relative to the patient anatomy included in the portion of the patient image shown in the window 152, 154, 156, for example, the first window 152.

The patient image may be a volumetric image, such as a CT image, and the windows 152, 154, 156 may each contain a "view" corresponding to the volumetric image. For example, each view may include at least a portion of a two-dimensional slice image derived from the volumetric image. Alternatively, the slice image may be a raw CT slice image upon which the volumetric image was generated. The views may alternatively include other two-dimensional images as long as a registration between the two-dimensional images and the volumetric images is known. In Figure 2, the views shown in the first and second windows 152, 154 each include a portion of a slice image which corresponds to slices of the volumetric image, and the view shown in the third window 156 includes a portion of a 3D model created based on the volumetric image. As such, the memory device may contain at least one slice image. The pose of the slice image(s) relative to the image coordinate system (and thus the volumetric image) may be known. The slice image(s) may be CT slice images and/or digitally reconstructed radiographs (DRRs).

The volumetric image may be stored in the memory device as a plurality of voxels defined relative to the image coordinate system. As such, the volumetric image may have a width along an x-axis of the image coordinate system, a depth along a y-axis of the image coordinate system, and a height along a z-axis of the image coordinate system.

The system 100 utilizes the controller 141 to control each window 152, 154, 156 of the GUI 150 by determining which view to show in each of the windows 152, 154, 156, and causing the GUI 150 to show said view(s). More specifically, the surgical instrument 172 is overlaid over the view (e.g. by overlaying a graphical representation of the surgical instrument 172 over the view), and the controller 141 can cause the GUI 150 to recenter at least one of the windows 152, 154, 156, such as the first window 152, on the surgical instrument 172. Recentering the view can be useful when the surgical instrument 172 is moved such that, for example, the end effector 174 is overlaid near or outside the bounds of the first window 152. If the view shown in the window 152, 154, 156 was not recentered, the user may have difficulty in discerning the position of the instrument 172 relative to the patient anatomy, especially if the instrument 172 is in a position such that it would no longer be displayed within the window 152, 154, 156.

The position of the surgical instrument 172 is tracked by the localizer 112. In some implementations, the localizer 112 tracks the surgical instrument 172 relative to a localizer coordinate system, the pose of which is known to the localizer 112. It is contemplated to instead track the instrument 172 relative to a world coordinate system (e.g. a coordinate defined relative to gravity). The localizer 112 may also track a position of the patient relative to the localizer coordinate system to allow the system 100 to determine if the patient has moved relative to the localizer 112. As such, the localizer 112 may track the positions of the surgical instrument 172 and the patient relative to the localizer coordinate system and, thus, relative to one another. The image coordinate system can be registered to the localizer coordinate system in any known way. For example, the pose of the patient relative to the localizer coordinate system may be determined according to a pose of a patient tracker coupled to the patient, and the pose of the patient anatomy in the volumetric image may be determined relative to the image coordinate system based on the pose of a patient tracker (not shown) which may have been coupled to the patient when the volumetric image was created and is present within the volumetric image. In some examples, the patient tracker may have been coupled to the patient during a CT scan of the patient upon which the volumetric image is based.

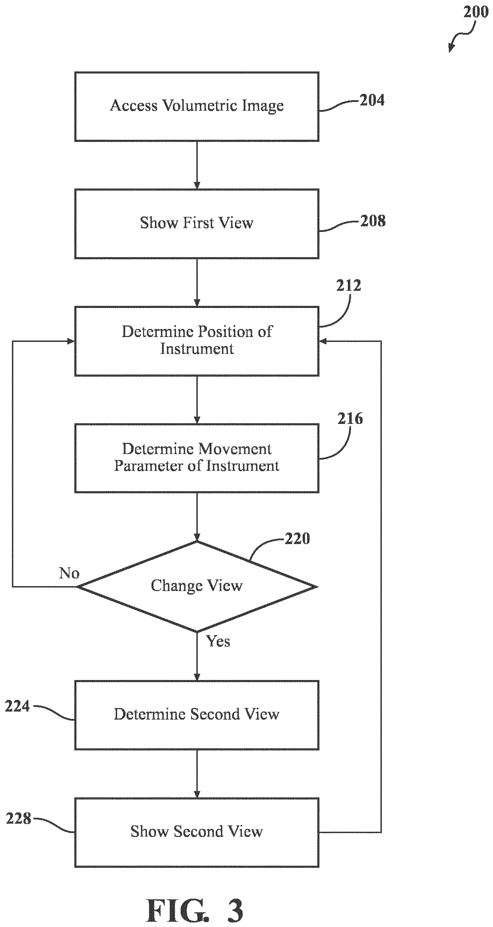

Referring now to Figure 3, a method 200 of controlling at least one of the windows 152, 154, 156 of the GUI 150 is shown. The method 200 is described as being carried out by the controller 141, but the method 200 may be performed by any capable computing device(s). The method 200 is meant to provide an automatic and predictable recentering function which changes the view shown in at least one of the windows 152, 154, 156 based on the position of the surgical instrument 172 as well as a movement parameter of the instrument 172. The method 200 is described below as changing the view shown in the first window 152, however, it is contemplated for the method 200 to apply to the second and/or third windows 154, 156 instead of the first window 152, or for the method 200 to be applied to all of the windows 152, 154, 156. In an implementation in which the method 200 is applied to multiple windows 152, 154, 156, the method 200 may be applied to one window 152, 154, 156 at a time (e.g. applied to one after another), applied to multiple windows 152, 154, 156 (but not all windows 152, 154, 156) at the same time, or continuously applied to each window 152, 154, 156.

Starting at step 204, the volumetric image is accessed in preparation for showing views within the first window 154 of the GUI 150 - the controller 141 may access (and retrieve) the volumetric image from the memory device Again, the method 200 is being described as being applied to the first window 152 but may be applied as described above. Subsequently, at step 208, a first view is shown within the first window 152. The first view may be a portion of at least one of the slice images which corresponds to a first portion of the volumetric image. In the illustrated implementation, the volumetric image (and slice images) includes a plurality of vertebrae of the patient, but the volumetric image may include any anatomy of the patient. The first view shown on the GUI 150 at step 208 may be based on surgical planning data, such as a planned starting point for a surgical operation, a planned position of a surgical implant, or the position of the surgical instrument 172 during/before step 208. Alternatively, the first view may be based on a default starting point and/or user preferences. The method then proceeds to step 212.

Once the method 200 reaches step 212, a loop of steps 212 through 228 is performed. The loop can be performed automatically or upon a triggering event, such as a user input. The loop can be performed continuously or at discrete intervals. At step 212, the position of the surgical instrument 172 is determined. The position of the surgical instrument 172 may be based on the pose of the tracking device 176 or other suitable means, such as using machine vision with a video camera. The position of the instrument 172 may be determined according to the position of the end effector 174, or even the tool center point thereof. At 216, the movement parameter of the surgical instrument 172 is determined. The movement parameter may include at least one of a displacement of the instrument 172, a speed of the instrument 172, an acceleration of the instrument 172, and/or a velocity of the instrument 170. In some implementations, the movement parameter is calculated to determine whether the instrument 172 is stationary or substantially so. In order to do so, the controller 141 may compare the speed of the instrument 172 against a threshold speed, an acceleration of the instrument 172 against a threshold acceleration, a velocity of the instrument 172 against a threshold velocity, and/or a displacement of the instrument 172 against a threshold displacement during a time period. In the last example, the displacement of the instrument 172 during the time period may be determined and the controller 141 may determine that the instrument 172 is stationary if the displacement during the time period is lower than the threshold displacement.

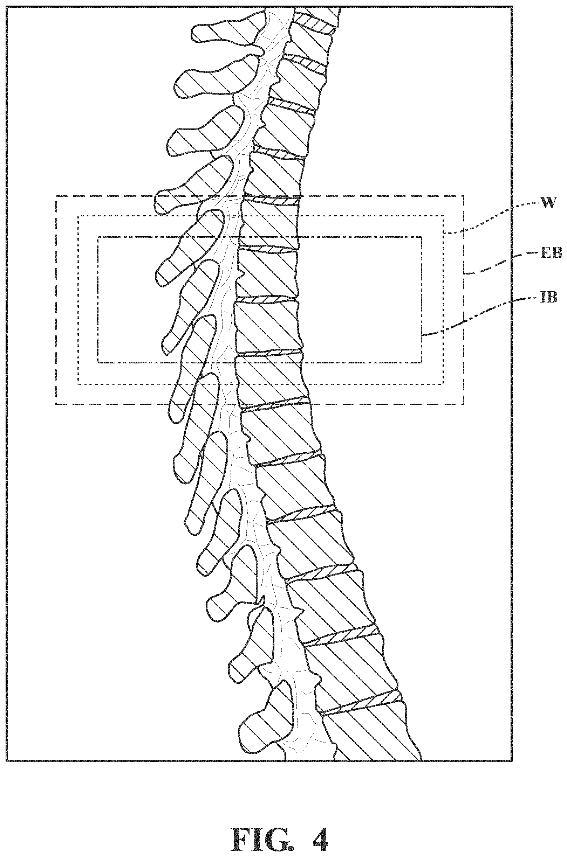

Once the controller 141 knows the position and movement parameter of the instrument 172, as determined at steps 212 and 216, the method 200 proceeds to step 220. At step 220 the controller 141 determines if the first view should be shown within the first window 152. More specifically, the controller 141 determines whether the view shown within the first window 152 should be shown on the display 120 based on the movement parameter of the instrument 172 and the position of the instrument 172 relative to the localizer coordinate system (or image/world coordinate system). If the first view should continue to be shown and does not need to be changed, the method 200 returns to step 212. On the other hand, if the controller 141 determines that the first view should no longer be shown, the method 200 continues to step 224. As mentioned above, in some implementations, the controller 141 may determine that the first view should no longer be shown if the instrument 172 is near the edge of the view and is stationary/substantially stationary. The determination performed at step 220 is further described references to Figures 4-7 below.

At 224, after determining that a different view should be shown in the first window 152, the controller 141 determines a second view to show within the first window 152. Similar to the first view, the second view may include a portion of at least one slice image which corresponds to a second portion of the volumetric image. After this determination, the method 200 proceeds to step 228, at which point the controller 141 causes the second view to be shown within the first window 152. In some implementations, the controller 141 may prompt an alert and/or notification as the second view is shown. The alert/notification is meant to inform the user that the second view is now being shown. The alert/notification may include displaying a banner notification on the GUI 150, a chime or other sound, a vibration of a footswitch or other user input device, and/or any other suitable notification type. After step 228, the method 200 returns to step 212. Although not explicitly shown in Figure 3, the second view may replace the first view such that the first view is no longer shown. Further, it will be understood that the loop of steps 212 to 228 may iteratively cause new views to replace the second, and subsequent, views. For example, a third view may replace the second view, a fourth view may replace the third view, and so on, as the loop iterates, such as during a navigated surgical operation.

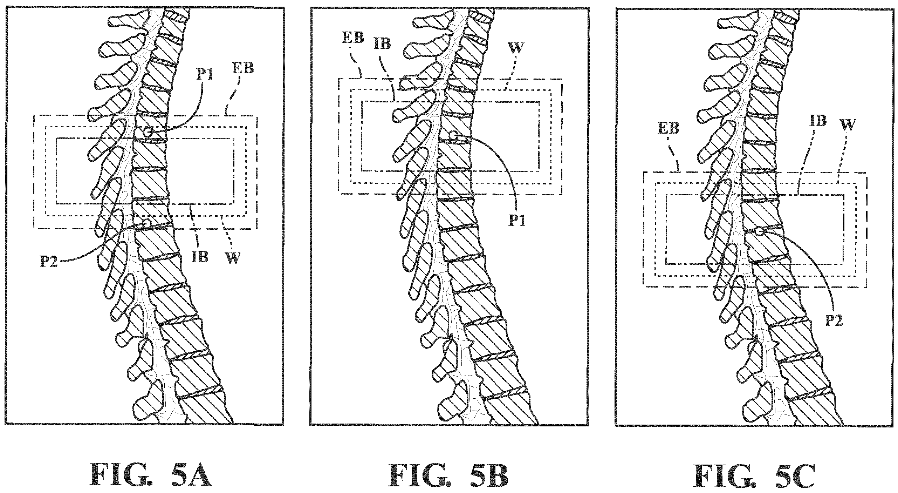

Referring to Figures 4-5C, a window frame W, an interior buffer IB, and an exterior buffer EB, each corresponding to at least one of the windows 152, 154, 156 of the GUI 150, are shown relative to a slice image which has been registered to the image coordinate system and the volumetric image. Like the description of the method 200 above, any of the following description directed to the first window 152 may also/alternatively be applied to the second and/or third windows 154, 156 of the GUI 150.

Referring specifically to Figure 4, the window frame W corresponding to the first window 152 is shown as a boundary overlaid on a two-dimensional image of patient anatomy. The window frame W encompasses the portion of the image which is shown within the first window 152 of the GUI 150. In Figure 4, the patient image is a two-dimensional slice image. Although the window frame W is shown as an actual boundary encompassing a portion of the image, this is merely for illustrative purposes. The window frame W is an abstract representation of the what the controller 141 understands as being shown within the first window 152, and the window frame W is meant to describe/illustrate this understanding. The window frame W and corresponding buffers IB, EB are also used to illustrate how the controller 141 understands the tracked position of the surgical instrument 172 relative to the first window 152.

The controller 141 may also know the relationship between the localizer coordinate system and the image coordinate system, which allows the controller 141 to translate the position of the instrument 172 relative to the localizer coordinate system into a position of the instrument 172 relative to the image coordinate system. This known relationship also allows the controller 141 to know the boundaries W, IB, EB relative to both the image coordinate system and the localizer coordinate system. As such, even though the window frame W and buffers IB, EB are illustrated as shapes overlaid on a two-dimensional image in the figures, the controller 141 may also understand these boundaries W, IB, EB in a three-dimensional sense. For example, the controller 141 may know the pose of these boundaries W, IB, EB relative to the image coordinate system.

As the volumetric image may be stored on the memory device as a plurality of voxels defined relative to the image coordinate system, the controller 141 may know the pose of the boundaries W, IB, EB relative to the volumetric image since the volumetric image is defined relative to the image coordinate system. In such an implementation, the window frame W may be understood as corresponding to a sub-volume of the volumetric image which is represented by the view shown within the first window 152. The window frame W may be based on, for example, an aspect ratio of the first window 152. Further, since the sub-volume corresponding to the window frame W in Figure 4 is known relative to image coordinate system, the pose of the sub-volume may be known relative to the localizer coordinate system. Thus, the position of the instrument 172 may be tracked relative to the window frame W and buffers IB, EB relative to the image coordinate system. Other tracking/registration methods for tracking the instrument 172 relative to the window frame W are contemplated.

The sub-volume may be defined between a set of coordinate bounds in at least the image coordinate system, including minimum x, y, and z-value coordinates and maximum x, y, and z-value coordinates. The distance between the minimum x-value and the maximum x-value is less than the width of the volumetric image, the distance between the minimum y-value and the maximum y-value is less than the depth of the volumetric image, and the distance between the minimum z-value and the maximum z-value is less than the height of the volumetric image. In the illustrated implementation, the width of the sub-volume is larger than the height of the sub-volume because the width of the window frame W is larger than the height of the window frame W. The controller 141 may adjust the geometry of the sub-volume based on the aspect ratio of the specific window 152, 154, 156 showing the portion of the volumetric image. The sub-volume may be similarly defined relative to the localizer coordinate system.

The controller 141 determines the position of the instrument 172 at step 212 of the method 200, along with the movement parameter as described above. Determining whether the view should be shown within the first window 152 may be based on the position of the instrument 172 relative to the window frame W and buffers IB, EB. In one implementation, the window frame W corresponds to the portion of the volumetric image represented by the view in the first window 152, an area/volume which is inside but near the edge of the window frame W is defined between the window frame W and the interior buffer IB, and an area/volume which is near but outside of the window frame W is defined between the window frame W and the exterior buffer EB. In such an implementation, the controller 141 may determine that the instrument 172 is near the edge of the window frame W by determining that, for example, a distal end of the end effector 174 is positioned between (1) the interior buffer IB and the window frame W, (2) the exterior buffer EB and the window frame W, and/or (3) the interior buffer IB and the exterior buffer EB. The controller 141 may additionally or alternatively determine the position of the distal end of the end effector 174 relative to the sub-volume of the volumetric image. As described in more detail below, the interior buffer IB may be represented by an interior sub-volume defined within the sub-volume corresponding to the window frame W, and the exterior buffer EB may be represented by an exterior sub-volume enclosing the sub-volume corresponding to the window frame W. Thus, the controller 141 may determine the position of the end effector 174 relative to the sub-volume, the interior sub-volume, and the exterior sub-volume. Any reference to the position of the surgical instrument 172 relative to the boundaries W, IB, EB may also include the position of the instrument 172 relative to the sub-volume, the interior sub-volume, and exterior sub-volume, respectively.

Referring specifically to Figures 5A-5C, two examples of steps 212 through 228 of the method 200 being carried out by the controller 141 are shown. Starting with Figure 5A, the window frame W and buffers IB, EB are shown along with two possible positions of the surgical instrument 172 - the positions of the surgical instrument 172 are shown as a first position P1 and a second position P2 in the illustrated implementation. In these examples, the surgical instrument 172 has been at one of the illustrated positions P1, P2 for a certain period of time such that the controller 141 determines that the instrument 172 is substantially stationary, such as at step 216 during the method 200.

In Figure 5A, first and second examples of steps 212 through 228 being carried out are shown. In the first example, the surgical instrument 172 is in first position P1, which is near an upper side of the window frame W and is between the window frame W and the interior buffer IB. During the method 200, the controller 141 determined that the surgical instrument 172 is at the first position P1 at step 212 and that the instrument 172 is substantially stationary at step 216. In the second example, the surgical instrument 172 is in second position P2, which is near a lower side of the window frame W and is between the window frame W and the exterior buffer EB. During both examples, the controller 141 determined that the surgical instrument 172 is at the first position P1, such as at step 212, and that the instrument 172 is substantially stationary, such as at step 216. Based on the instrument 172 being in the respective positions P1, P2 and being substantially stationary, the controller 141 has determined that the view shown in the first window 152 should be changed/updated, such as at step 220 of the method 200.

In Figures 5B and 5C, the view determinations carried out by the controller 141 during of the first and second examples is shown. Figure 5B represents the second view determined by the controller 141 due to the surgical instrument 172 being in the first position P1, and Figure 5C represents the second view determined by the controller 141 due to the instrument 172 being in the second position P2. As shown in the figures, the window frame W (along with the buffers IB, EB) has been centered on the first and second positions P1, P2, respectively.

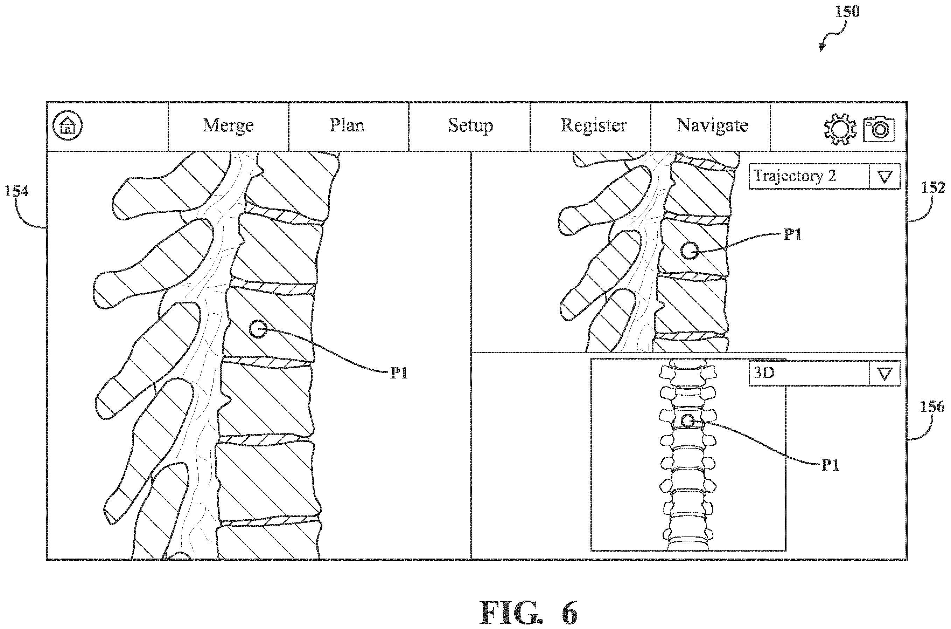

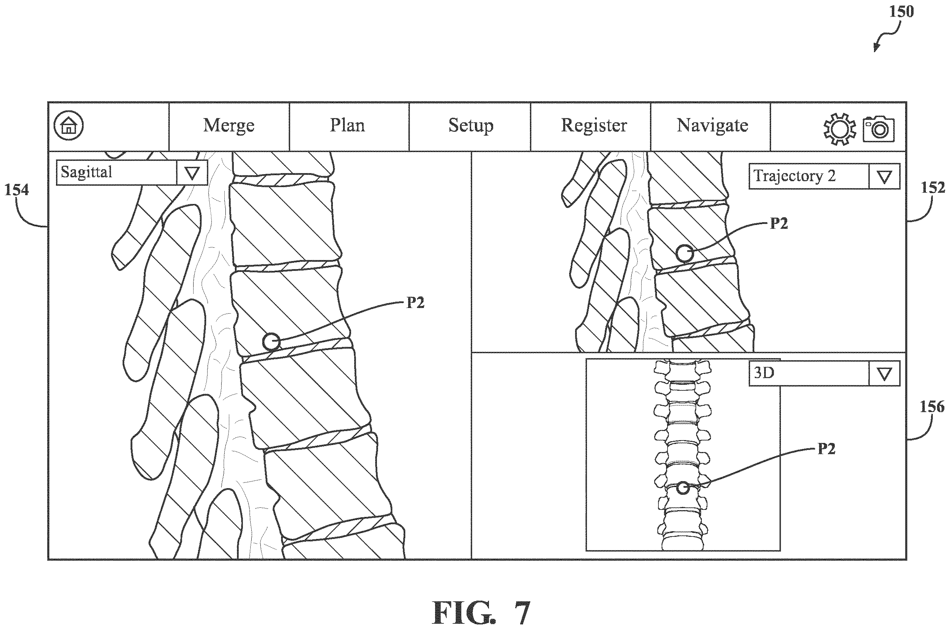

Referring specifically to Figures 6 and 7, the views resulting from the first and second examples are shown within various windows 152, 154, 156 of the GUI 150. The controller 141 has caused the GUI 150 to display these views, such as at step 228 of the method 200. In these figures, the views shown in the first and second windows 152, 154 have been changed based on the position (and movement parameter) of the instrument 172. The third window 156 has not been changed to illustrate an example in which the controller 141 only causes the first and second windows 152, 154 to change their views according to the method 200. Further, the third window 156 is configured to display a 3D model of the patient anatomy in the illustrated implementation, while the first and second windows 152, 154 are configured to display two dimensional views of the patient anatomy (e.g. an individual slice of the CT image, a digitally reconstructed radio graph (DRR), or the like). It is also contemplated to change the view of the 3D model based on the position and movement parameter of the instrument 172 as noted above. The positions of the instrument P1, P2 are overlaid over the views shown in each of the windows 152, 154, 156.

In Figure 6, the view corresponding to the first example and Figure 5B is shown in the first window 152. The view shown in the second window 154 has been similarly updated. In comparison to the view of the patient anatomy shown within the first and second windows 152, 154 in Figure 2, the views shown in Figure 6 are positioned higher up along the spine of the patient because the surgical instrument 172 was near the upper side of the window frame W. The views are also centered on the first position P1, but it is contemplated to determine the view (i.e. determine the updated window frame W) as being offset from the position of the instrument 172 by a specific distance and direction.

In Figure 7, the view corresponding to the second example and Figure 5C is shown in the first window 152. The view shown in the second window 154 has been similarly updated. In comparison to the view of the patient anatomy shown within the first and second windows 152, 154 in Figure 2, the views shown in Figure 7 are positioned lower down along the spine of the patient because the surgical instrument 172 was near the lower side of the window frame W. The views are also centered on the second position P2.

Now referring to figures generally, the term "view" is used above to describe a portion of an image depicting patient anatomy which is shown within the windows 152, 154, 156 of the GUI. That being said, the system 100 has also been described as determining the position and movement parameter of the surgical instrument 172 relative to the (three-dimensional) localizer and/or image coordinate systems. Further, when describing how the system 100 determines a new view to display on the GUI 150, such as at step 224 of the method 200, the new view determination was described generally. The meaning of view as used herein, as well as the new view determination, will now be described in more detail.

Regarding views shown on the GUI 150, the word view is used herein to describe what is shown within the windows 152, 154, 156 of the GUI 150. In one implementation, the view is a portion of a two-dimensional image of the patient anatomy. For example, the view may be a portion of a DRR or a CT image slice. In another implementation, the view may be a portion of a volumetric image of the patient anatomy. In yet another implementation, the view may be a portion of a 3D model of the patient anatomy, such as a 3D model created from the volumetric image of the patient anatomy via a process such as segmentation.

Regarding new view determinations, the system 100 may determine a new view to display on the GUI 150, such as at step 224 of the method 200, based on the position of the surgical instrument 172 along with movement parameters thereof - and both the position and movement parameters of the instrument 172 are tracked by the localizer 112 relative to the localizer coordinate system, which is a three-dimensional coordinate system. Thus, in implementations where the view shown on the GUI 150 includes a two-dimensional image, such as a DRR or CT image slice, the system 100 may actually track the position of the instrument 172 relative to the localizer coordinate system and not relative to the image coordinate system. For example, referring back to Figure 5A, the first and second positions P1, P2 are illustrated as points on a two-dimensional image representative of the patient anatomy. Although these points are illustrated as such, the system 100 may understand these positions P1, P2 as locations relative to the three-dimensional localizer coordinate system. The system 100 may determine (or otherwise know based on previous registration of the image coordinate system to the localizer coordinate system) the location/pose of the window frame W and buffers IB, EB relative to the localizer coordinate system. As such, the instrument 172 may be tracked relative to three-dimensional versions of the window frame W and buffers IB, EB.

In one example, the view is a portion of a two-dimensional CT image slice of the spine of the patient from a sagittal perspective, similar to the illustrated implementation. Further, the system 100 may determine/know the position of each pixel of the image relative to the localizer coordinate system such that the system 100 understands the bounds of the first window 152 (i.e. the window frame W and/or sub-volume corresponding thereto) relative to the localizer coordinate system. In such an example, the location of each pixel is constrained to a plane defined relative to the localizer coordinate system. The locations of the window frame W and buffers IB, EB are also constrained to the plane in this example. The system 100 then tracks the position and movement of the surgical instrument 172 relative to this plane. Thus, the system 100 may determine that the instrument 172 (e.g. the distal end of the end effector 174) is positioned on the plane, is between the interior buffer IB and the window frame W as defined relative to the localizer coordinate system, and has been stationary for the last 3 seconds. At this point, the system 100 may determine a new view based on the position of the instrument 172.

Referring back to Figures 5A-7 and continuing the above example, determining the new view is also based on the position of the surgical instrument 172 relative to the localizer coordinate system. This may be done by treating the position of the instrument 172 as the center of focus of the new view and then calculating new positions of the window frame W and buffers IB, EB relative to this center. The system 100 treats the position of the instrument 172 as the center of focus of the new view since the new view may not be a view of a portion of the volumetric image but instead be a portion of a two-dimensional image, the center of which corresponds to a point in the image coordinate system and thus the volumetric image. Some parts of the present description refer to calculations/determinations relative to the image coordinate system, while other parts are described relative to the localizer coordinate system. These coordinate systems may be registered relative to one another by the system 100, and it will be appreciated that the calculations/determinations performed relative to one of these coordinate systems may be performed relative the either of these coordinate systems.

Once the center of focus of the new view is determined, a sub-volume may be defined surrounding the center of focus such that the center of focus of the new view become a centroid of the sub-volume. The sub-volume is defined based on the type of view (e.g. sagittal, lateral, etc.) and the aspect ratio of the specific window 152, 154, 156 of the GUI 150 in which the view is shown. Continuing the above example, the view is a sagittal view of the patient anatomy, and the first window 152 has a rectangular shape with a larger width than height. The resultant sub-volume is therefore a rectangular prism centered on the centroid. Further, the sub-volume may have a depth and/or be substantially planar. For example, if the system 100 is configured to display a sagittal view, the sub-volume may be a plane constrained to a sagittal slice of the volumetric image. In another example, the system 100 is configured to display one or more views that intersects the planned trajectory of a screw to be inserted into the patient anatomy.

After the sub-volume is calculated/determined, the views are determined based on the pose of the sub-volume relative to the image coordinate system. As described above, the volumetric image may be understood as a plurality of voxels defined relative to the image coordinate system. As such, the sub-volume may be understood as being defined relative to the volumetric image itself. The centroid of the sub-volume may also be understood as being defined relative to the volumetric image as well. The pose of the sub-volume, however, may also need to be determined. More specifically, although the system 100 may know the center of focus of the new view, the system 100 may still need to determine how the view should be oriented relative to this center of focus. In other words, the system 100 may need to determine the pose of the window frame W, not just the size and aspect ratio of the window frame. With this information, the system 100 can determine the appropriate two-dimensional image (and portion thereof) based on pose of the sub-volume relative to the volumetric image.

The system 100 may determine the pose of the sub-volume by determining a perspective reference point in the image coordinate system, determining a view axis defined as a line extending between the perspective reference point and the centroid of the sub-volume, and aligning at least one face of the sub-volume such that the face of the sub-volume is orthogonal to the line which extends between the perspective reference point and the centroid of the sub-volume. The perspective reference point may be based on the desired perspective of the view and the centroid of the sub-volume. For example, in the illustrated example of Figures 5A-5C, the views are based on a sagittal image of the patient anatomy. Since sagittal slices are parallel to a yz-plane of the image coordinate system, the view will need to be substantially parallel to the yz-plane. As such, the perspective reference point may be determined as a point in the image coordinate system which is displaced from the centroid of the sub-volume in the direction of the x-axis.

In some implementations, the view axis is calculated based on a perspective vector instead. The perspective vector may be a vector extending/pointing from the centroid of the sub-volume in a direction, the direction being based on the desired view. For example, where the view is a sagittal view, the perspective vector may extend such that the view axis is perpendicular to the yz-plane and/or parallel to the x-axis of the image coordinate system.

Once the system 100 knows the centroid of the sub-volume and the view axis, the system 100 may also determine the orientation/rotation of the sub-volume about the view axis. The orientation of the sub-volume is directly related to the orientation of the view. For example, looking to the example shown in Figures 5A-5B, the orientation of the window frame W and buffers IB, EB is the same as the orientation of the view. Initially, the sub-volume may be oriented such that the depth of the sub-volume extends along the view axis. Next, the direction in which the width and height of the sub-volume span are defined to complete the pose of the sub-volume. Although both the width and height of the sub-volume need to be defined in order to define the pose of the sub-volume, only one of the two is required to be determined since the height and width (i.e. x/y axes) are known to be orthogonal to one another. In some implementations, such as the illustrated implementation, the height of the sub-volume is defaulted to be parallel to the y-axis of the image coordinate system. This ensures that the view of the patient anatomy is in an orientation which is familiar to the user.

Although the system 100 may need to determine the pose of the sub-volume in order to determine the view to show on the GUI 150, the orientation of the sub-volume may partially fixed when calculating/determining new views. For example, the window frames W in Figures 5A-5C (i.e. the sub-volumes) may be translated along the yz-plane of the image coordinate system relative to one another, but not translated along the x-axis or rotated about the y and z axes. This allows the system 100 to calculate a single DRR or choose a single CT image slice, and then display different portions of the DRR/CT image slice without needing to calculate different view axes.

Further, the sub-volume has been described as being representative of the view shown on the GUI 150. Although this is true, the system 100 may define an interior sub-volume inside of the sub-volume which is representative of the interior buffer IB, and an exterior sub-volume surrounding the sub-volume which is representative of the exterior buffer EB. The interior sub-volume and the exterior sub-volume may be centered on the centroid of the sub-volume and with poses substantially similar to the sub-volume. Thus, although the user may simply see the surgical instrument 172 as being near the edge of the window 152, 154, 156, the system 100 may know that the instrument 172 is positioned relative to the localizer coordinate system and in a space defined between the sub-volume (i.e. window frame W) and one of the interior sub-volume (i.e. interior buffer IB) and exterior sub-volume (i.e. exterior buffer EB).

In any of the above implementations, the system 100 may also track the position relative to the sub-volume and/or window frame without defining the interior/exterior sub-volumes and/or buffers IB, EB, Instead, the system 100 may define a threshold distance and consider the instrument 172 to be near the bounds of the sub-volume, and thus the bounds of the window frame W, based on a comparison of the position of the instrument 172 and the bounds of the sub-volume. For example, the system 100 may determine the distance between the position of the instrument 172 and the coordinates defining the sub-volume and compare the distance against a threshold distance. If the distance is lower than the threshold distance, the system 100 may determine that the view should be changed, such as at step 220. In some implementations, the system 100 may define an interior threshold distance and an exterior threshold distance, determine whether the surgical instrument 172 is inside the sub-volume or outside the sub-volume, determine the distance between the instrument 172 and the bounds of the sub-volume, and then compare the distance to the one of the interior and exterior threshold distances based on whether the instrument 172 is inside/outside the sub-volume. For example, the system 100 may determine that the view should be changed if the instrument 172 is within the sub-volume and the distance between the instrument 172 and the bounds of the sub-volume is lower than the interior threshold distance. In such an example, the system 100 may also determine that the view should be changed if the instrument 172 is outside the sub-volume and the distance between the instrument 172 and the bounds of the sub-volume is lower than the exterior threshold distance. The distance between the instrument 172 and the bounds of the sub-volume may be determined as the distance between the instrument 172 and at least one coordinate of the set of coordinate bounds, such as the coordinate which is closes to the position of the instrument 172.

Several implementations have been discussed in the foregoing description. However, the implementations discussed herein are not intended to be exhaustive or limit the invention to any particular form. Modifications and variations are possible in light of the above teachings and may be practiced otherwise than as specifically described.

## Claims

1. A surgical navigation system (110) comprising:
a localizer (112) configured to track a position of an instrument (172);
a memory device containing:
a volumetric image defined relative to a known coordinate system and including a width along an x-axis of the known coordinate system, a depth along a y-axis of the known coordinate system, and a height along a z-axis of the known coordinate system, and
at least one slice image derived from the volumetric image;
a display (120); and
a controller (141) in communication with the localizer (112), the display (120), and the memory device, the controller (141) configured to:
show a view of the at least one slice image which corresponds to a first sub-volume of the volumetric image on the display (120),
determine a position of the instrument (172) relative to the known coordinate system,
determine a movement parameter of the instrument (172),
determine that the view of the at least one slice image which corresponds to the first sub-volume should no longer be shown on the display (120) based on the movement parameter of the instrument (172) and the position of the instrument (172) relative to the known coordinate system,
determine a second sub-volume of the volumetric image based on the position of the instrument (172) relative to the known coordinate system, and
show a view of the at least one slice image which corresponds to the second sub-volume of the volumetric image on the display (120).

2. The surgical navigation system (110) of claim 1, wherein:
the first sub-volume is defined between a first set of coordinate bounds; and
the first set of coordinate bounds includes:
a minimum x-value coordinate,
a minimum y-value coordinate,
a minimum z-value coordinate,
a maximum x-value coordinate,
a maximum y-value coordinate, and
a maximum z-value coordinate.

3. The surgical navigation system (110) of claim 2, wherein:
a distance between the minimum x-value and the maximum x-value is less than the width of the volumetric image;
a distance between the minimum y-value and the maximum y-value is less than the depth of the volumetric image; and
a distance between the minimum z-value and the maximum z-value is less than the height of the volumetric image.

4. The surgical navigation system (110) of any one of claims 2-3, wherein the controller (141) is further configured to show the view of the at least one slice image which corresponds to the second sub-volume of the volumetric image based on the movement parameter of the instrument (172) and the first set of coordinate bounds.

5. The surgical navigation system (110) of any one of claims 2-4, wherein the controller (141) is further configured to:
determine a distance between the position of the instrument (172) and at least one coordinate of the first set of coordinate bounds; and
show the view of the at least one slice image which corresponds to the second sub-volume of the volumetric image based on the movement parameter of the instrument (172) and the distance between the position of the instrument (172) and at least one coordinate of the first set of coordinate bounds.

6. The surgical navigation system (110) of claim 5, wherein the controller (141) is further configured to:
compare the distance between the position of the instrument (172) and at least one coordinate of the first set of coordinate bounds against a threshold distance; and
show the view of the at least one slice image which corresponds to the second sub-volume of the volumetric image based on the movement parameter of the instrument (172) and comparison of the distance between the position of the instrument (172) and at least one coordinate of the first set of coordinate bounds and the threshold distance.

7. The surgical navigation system (110) of any one of claims 5-6, wherein the controller (141) is further configured to:
determine that the position of the instrument (172) is outside of the first sub-volume;
compare the distance between the position of the instrument (172) and at least one coordinate of the set of coordinate bounds against an exterior threshold distance; and
show the view of the at least one slice image which corresponds to the second sub-volume of the volumetric image based on the movement parameter of the instrument (172) and the distance between the position of the instrument (172) and at least one coordinate of the set of coordinate bounds being below the exterior threshold distance.

8. The surgical navigation system (110) of any one of claims 5-7, wherein the controller (141) is further configured to:
determine that the position of the instrument (172) is inside of the first sub-volume;
compare the distance between the position of the instrument (172) and at least one coordinate of the first set of coordinate bounds against an interior threshold distance; and
show the view of the at least one slice image which corresponds to the second sub-volume of the volumetric image based on the movement parameter of the instrument (172) and the distance between the position of the instrument (172) and at least one coordinate of the first set of coordinate bounds being below the interior threshold distance.

9. The surgical navigation system (110) of any preceding claim, wherein the movement parameter includes at least one of:
a displacement of the instrument (172);
a speed of the instrument (172);
an acceleration of the instrument (172); and
a velocity of the instrument (172).

10. The surgical navigation system (110) of any preceding claim, wherein:
the movement parameter includes a displacement of the instrument (172) during a first time period;
the controller (141) is configured to:
compare the displacement of the instrument (172) during the first time period to a threshold displacement; and
show the view of the at least one slice image which corresponds to the second sub-volume of the volumetric image on the display (120) based on the position of the instrument (172) relative to the first sub-volume of the volumetric image and comparison of the displacement of the instrument (172) during the first time period to the threshold displacement.

11. The surgical navigation system (110) of any preceding claim, wherein to show the view of the at least one slice image which corresponds to the second sub-volume on the display (120), the controller (141) is configured to:
determine a perspective reference point in the known coordinate system,
determine a view axis defined as a line between the perspective reference point and a centroid of the second sub-volume,
determine which slice image of the at least one slice image corresponds to the view axis,
determine which portion of the determined slice image corresponds to the second sub-volume, and
show the determined portion of the determined slice image.

12. The surgical navigation system (110) of any preceding claim, wherein to show the view of the at least one slice image which corresponds to the second sub-volume on the display (120), the controller (141) is configured to:
determine a perspective vector in the known coordinate system which includes a direction,
determine a view axis defined as a line extending from a centroid of the second sub-volume and in the direction of the perspective vector,
determine which slice image of the at least one slice image corresponds to the view axis,
determine which portion of the determined slice image corresponds to the second sub-volume, and
show the determined portion of the determined slice image.

13. The surgical navigation system (110) of any preceding claim, wherein:
to determine the second sub-volume, the controller (141) is configured to determine a centroid for the second sub-volume, and the controller (141) determines the centroid of the second sub-volume based on:
the position of the instrument (172) relative to the known coordinate system; or
a coordinate point offset from the position of an instrument center point of the instrument (172) by a predefined distance.

14. A computer program product comprising instructions, which when executed by one or more processors, are configured to:
access a volumetric image from a memory device, the volumetric image being defined relative to a known coordinate system;
access at least one slice image from the memory device, the at least one slice image being derived from the volumetric image;
control a display (120) to show a view of the at least one slice image which corresponds to a first sub-volume of the volumetric image on the display (120);
determine a position of an instrument (172) relative to the known coordinate system;
determine a movement parameter of the instrument (172);
determine that the view of the at least one slice image which corresponds to the first sub-volume should no longer be shown on the display (120) based on the movement parameter of the instrument (172) and the position of the instrument (172) relative to the known coordinate system;
determine a second sub-volume of the volumetric image based on the position of the instrument (172) relative to the known coordinate system; and
control the display (120) to show a view of the at least one slice image which corresponds to the second sub-volume of the volumetric image on the display (120).

15. A computer-implemented method of displaying an instrument (172) relative to a volumetric image of a patient anatomy on a display (120), the computer-implemented method comprising:
accessing (204) the volumetric image from a memory device, the volumetric image being defined relative to a known coordinate system;
accessing at least one slice image from the memory device, the at least one slice image being derived from the volumetric image;
controlling (208) the display (120) to show a view of the at least one slice image which corresponds to a first sub-volume of the volumetric image on the display (120);
determining (212) a position of the instrument (172) relative to the known coordinate system;
determining (216) a movement parameter of the instrument (172);
determining (220) that the view of the at least one slice image which corresponds to the first sub-volume should no longer be shown on the display (120) based on the movement parameter of the instrument (172) and the position of the instrument (172) relative to the known coordinate system;
determining (224) a second sub-volume of the volumetric image based on the position of the instrument (172) relative to the known coordinate system; and
controlling (228) the display (120) to show a view of the at least one slice image which corresponds to the second sub-volume of the volumetric image on the display (120).
